(19) Europäisches Patentamt / European Patent Office / Office européen des brevets

(11) **EP 4 477 211 A1**

(12) **EUROPEAN PATENT APPLICATION**
published in accordance with Art. 153(4) EPC

(43) Date of publication:
**18.12.2024 Bulletin 2024/51**

(21) Application number: 23752834.4

(22) Date of filing: **06.02.2023**

(51) International Patent Classification (IPC):
**A61K 8/06** *(2006.01)*     **A61K 8/60** *(2006.01)*
**A61K 8/73** *(2006.01)*

(52) Cooperative Patent Classification (CPC):
**A23L 29/30; A61K 8/06; A61K 8/60; A61K 8/73;**
A23C 9/154; A23C 13/14; A23L 27/00

(86) International application number:
**PCT/JP2023/003838**

(87) International publication number:
**WO 2023/153370 (17.08.2023 Gazette 2023/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.02.2022 PCT/JP2022/005105**

(71) Applicant: **Showa Sangyo Co., Ltd.**
**Tokyo 101-8521 (JP)**

(72) Inventors:
• **KAWANO Atsushi**
 **Tokyo 101-8521 (JP)**
• **SHINAGAWA Yuya**
 **Tokyo 101-8521 (JP)**

(74) Representative: **Forrest, Stuart**
**WP Thompson**
**138 Fetter Lane**
**London EC4A 1BT (GB)**

(54) **OIL-IN-WATER TYPE EMULSION COMPOSITION, AND COSMETIC USING SAME**

(57) [Summary]

[Problems] To provide an oil-in-water type emulsion composition for cosmetics that can realize reduction in the use of synthetic additives such as synthetic emulsifiers, synthetic thickeners, synthetic texture improving agents, etc.

[Solution] This technique provides an oil-in-water type emulsion composition that contains a starch decomposition product having a content of a glucose polymerization degree (DP) of 8 to 19 of 32% or more and a content of a glucose polymerization degree (DP) of 20 or more of 30% or less, water, and an oily component, in which the ratio of the starch decomposition product/water is 0.05 or more.

**Description**

Technical field

The present technique relates to an oil-in-water type emulsion composition and a cosmetic using the oil-in-water type emulsion composition.

Background Art

In recent years, as a countermeasure against allergies, techniques for preparing oil-in-water type emulsion compositions without using milk or eggs are being developed. For example, Patent Document 1 discloses an ice cream-like emulsion composition having a smooth food texture in which the increase and growth of ice crystals during the freezing and storage period are suppressed even when substantially no protein is included, by containing water, oils and fats, and cyclodextrin, as well as trehalose and dextrin.

Furthermore, Patent Document 2 discloses an ice cream-like aerated emulsion composition that has a soft and smooth food texture characteristic of ice cream even when substantially no protein is included, by containing more than 52% by mass of water, less than 23% by mass of oils and fats, as well as cyclodextrin and a water-soluble gelling agent.

Furthermore, Patent Document 3 discloses a mayonnaise-like dressing that has physical properties comparable to mayonnaise even though it does not contain proteins such as eggs, by adding an emulsifier to a mixture obtained by mixing water, oils and fats, cyclodextrin, and a water-soluble gelling agent, and further mixing the obtained mixture.

These oil-in-water type emulsion compositions use specific saccharides and the like without using milk or eggs, and saccharides are also used in various applications in the cosmetics field. For example, Patent Document 4 discloses a technique for producing cosmetics with excellent texture and moisturizing properties during use by using enzymatically synthesized $\alpha$-1,4-glucan with an average molecular weight of 100,000 or more and 6,000,000 or less.

Furthermore, Patent Document 5 discloses a two-component sheet-like cosmetic pack in which a water-insoluble gel sheet made of amylose is impregnated with a serum, and it is described that this sheet-like cosmetic pack is flexible and strong, easy to use, does not cause dripping even if the liquid content is high, and has a sebum removal effect due to the inclusion function of amylose.

Citation List

Patent Literature

Patent Literature 1: JP 2021-52739 A
Patent Literature 2: JP 2021-122269 A
Patent Literature 3: JP 2021-19525 A
Patent Literature 4: JP 2004-315481 A
Patent Literature 5: JP 2005-213176 A

Summary of Invention

Technical Problem

In addition to the aforementioned allergy countermeasures, people are becoming more health-conscious, and there is a desire to reduce the amount of synthetic additives used in the food and cosmetic fields. Various additives made from natural resources are being developed, but the reality is that many of them have problems in their actions and effects.

Therefore, the main purpose of this technique is to provide an oil-in-water type emulsion composition that can reduce the use of synthetic additives such as synthetic emulsifiers, synthetic thickeners, synthetic texture improving agents, and the like.

Solution to Problem

This technique first provides an oil-in-water type emulsion composition comprising:

a starch decomposition product having
a content of a glucose polymerization degree (DP) of 8 to 19 of 32% or more and

a content of a glucose polymerization degree (DP) of 20 or more of 30% or less;
water; and
an oily component;
wherein the ratio of the starch decomposition product/water is 0.05 or more.

The starch decomposition product having an iodine color value of 0.35 or more can be used in the oil-in-water type emulsion composition according to the present technique.

The oil-in-water type emulsion composition according to the present technique can contain 1.5 to 30% by mass of the starch decomposition product.

Furthermore, the oil-in-water type emulsion composition according to the present technique can contain 15 to 65% by mass of the oily component.

The oil-in-water type emulsion composition according to the present technique can contain an emulsifier.

In this case, the oil-in-water type emulsion composition according to the present technique can contain 0.5 to 5% by mass of the emulsifier.

Further, the oil-in-water type emulsion composition according to the present technique can contain a cyclic oligosaccharide as an emulsifier. Furthermore, $\alpha$-cyclodextrin can be used as the cyclic oligosaccharide.

Next, the present technique provides a cosmetic using the oil-in-water type emulsion composition according to the present technique.

Description of Embodiments

Preferred embodiments for carrying out the present technique will be described below. Note that the embodiment described below is an example of a typical embodiment of the present technique, and the scope of the present technique is not to be interpreted narrowly by this.

1. Oil-in-water type emulsion composition

(1) Blending starch decomposition product and water

The oil-in-water type emulsion composition according to the present technique is characterized by containing a specific starch decomposition product, water, and an oily component, and having a starch decomposition product/water ratio of 0.05 or more. In blending a starch decomposition product and water, the effects of the present technique can be exhibited as long as the ratio of starch decomposition product/water is 0.05 or more, but the lower limit of starch decomposition product/water is preferably 0.1 or more, more preferably 0.2 or more. By setting the ratio of starch decomposition product/water to 0.05 or more, it is possible to obtain an oil-in-water type emulsion composition with appropriate hardness and viscosity without using synthetic additives, and even when synthetic additives are used, their amounts can be reduced, as shown in the examples below.

The upper limit of starch decomposition product/water is not particularly limited as long as it does not impair the effects of the present technique, and can be freely set depending on the intended use, but for example, it is 2.0 or less, preferably 1.5 or less, and more preferably 1.3 or less. By setting the upper limit of starch decomposition product/water within this range, it is possible to obtain an oil-in-water type emulsion composition with a good emulsification state and appropriate hardness and viscosity without using synthetic additives, and even if synthetic additives are used, their amounts can be reduced.

(2) Viscosity

As shown in the examples below, the oil-in-water type emulsion composition according to the present technique has the characteristic that it is solid or has high viscosity when left to stand, and has low viscosity when the viscosity is measured at a high rotation speed. Due to this characteristic, for example, the oil-in-water type emulsion composition according to the present technique can contribute to stabilizing the quality by being solid or having high viscosity during storage, while the viscosity can be reduced by applying some external force during use, improving the use feeling. To give a more specific example, when the oil-in-water type emulsion composition according to the present technique is used in a cosmetic, it can maintain high quality by being solid or having high viscosity during storage, while the viscosity is reduced when applied to the skin or hair during use, improving the use feeling, and improving adhesion to the skin or hair and the ability to follow the unevenness of the skin or hair.

The specific viscosity of the oil-in-water type emulsion composition according to the present technique is not particularly limited as long as it does not impair the effects of the present technique, and can be freely set according to the intended use. The lower limit of the viscosity at 25°C and a rotational speed of 2/s is, for example, 4 Pa·s or more, preferably 6 Pa·s or more, and more preferably 7 Pa·s or more. By setting the lower limit of the viscosity at 25°C and a rotational speed of 2/s within this range, the quality retention of the oil-in-water type emulsion composition during storage can be improved.

The upper limit of the viscosity of the oil-in-water type emulsion composition according to the present technique at 25°C and a rotational speed of 2/s is, for example, 2000 Pa·s or less, preferably 1000 Pa·s or less, more preferably 800 Pa·s or less, and even more preferably is 500 Pa·s or less. By setting the upper limit of the viscosity at 25°C and a rotational speed of 2/s within this range, the use feeling of the oil-in-water type emulsion composition at the beginning of use can be improved. For example, when using the oil-in-water type emulsion composition according to the present technique in cosmetics, if the viscosity is too high, problems may occur such as difficulty in taking out cosmetics from storage containers using fingers or spatulas, or when taking out cosmetics from storage containers such as tubes, but, by setting the upper limit of the viscosity at 25°C and a rotation speed of 2/s within this range, it is possible to improve the workability when taking out from the storage container.

More specifically, when used in liquid cosmetics such as lotions, emulsions, hair lotions, and cleansing oils, the viscosity is preferably 4 to 20 Pa·s, more preferably 6 to 18 Pa·s, and still more preferably 7 to 16 Pa·s. When used in cream cosmetics such as hand creams and body creams, and in gel cosmetics, the viscosity is preferably 10 to 2000 Pa·s, more preferably 13 to 1500 Pa·s, and even more preferably 16 to 1000 Pa·s.

The lower limit of the viscosity of the oil-in-water type emulsion composition according to the present technique at 25°C and a rotation speed of 200/s is, for example, 0.05 Pa s or more, preferably 0.1 Pa·s or more, and more preferably 0.15 Pa s or more. The upper limit of the viscosity at 25°C and a rotation speed of 200/s is, for example, 4.0 Pa·s or less, preferably 3.5 Pa·s or less, more preferably 3.0 Pa·s or less, and even more preferably 2.5 Pa·s or less. By setting the upper limit of the viscosity at 25°C and a rotation speed of 200/s within this range, the use feeling can be improved when using the oil-in-water type emulsion composition. For example, the workability during application to skin or hair can be improved. In addition, by setting the lower limit of the viscosity at 25°C and a rotation speed of 200/s within this range, for example, when the oil-in-water type emulsion composition according to the present technique is used in a cosmetic, the adhesion to skin or hair can be improved.

In the oil-in-water type emulsion composition according to the present technique, the difference between the viscosity at 25°C and a rotation speed of 2/s and the viscosity at 25 °C and a rotation speed of 200/s is also not particularly limited as long as it does not impair the effects of the present technique, and can be freely set according to the intended use. The lower limit of the difference between the viscosity at 25°C and a rotation speed of 2/s and the viscosity at 25°C and a rotation speed of 200/s is, for example, 6.0 Pa s or more, preferably 8.0 Pa s or more, more preferably is 10 Pa·s or more, even more preferably 15 Pa·s or more, and particularly preferably 30 Pa·s or more. Further, the upper limit of the difference between the viscosity at 25°C and a rotation speed of 2/s and the viscosity at 25°C and a rotation speed of 200/s is, for example, 950 Pa·s or less, preferably 500 Pa·s or less, more preferably 300 Pa·s or less, and even more preferably 200 Pa·s or less. By setting the difference between the viscosity at 25°C and a rotational speed of 2/s and the viscosity at 25°C and a rotational speed of 200/s within this range, a moderate change in the use feeling from the beginning of use to during use can be caused, and it is also possible to make the user experience the change in the use feeling depending on the usage situation.

In the present technique, the "viscosity" is a value measured by setting 25 mm parallel plates at a gap of 1 mm on a rheometer MCR102 (manufactured by Anton Paar).

The oil-in-water type emulsion composition according to the present technique can also be allowed to contain an emulsifier such as a cyclic oligosaccharide and the like, and other components, as necessary, in addition to a specific starch decomposition product, water, and an oily component. Each component will be described in detail below.

(3) Starch decomposition product

The starch decomposition product used in this technique has a concept including those obtained by decomposing (saccharifying) starch raw materials, for example, starches (ground starch) such as corn starch, waxy corn starch, high amylose corn starch, rice starch, wheat starch, and the like, rhizome or root-derived starches (underground starch) such as potato starch, tapioca starch, sweet potato starch, and the like, or processed starches obtained by subjecting these starches to physical or chemical processing singly or in combination; and also those obtained by further converting them into sugar alcohols. The starch raw material used is not particularly limited, and any starch raw material can be used.

The compositional characteristic of the starch decomposition product used in the present technique is that the content of a glucose polymerization degree (hereinafter, referred to as "DP") of 8 to 19 is 32% or more, and the content of a DP of 20 or more is 30% or less. The starch decomposition product used in the present technique contains a large amount of high molecular weight oligosaccharide components and low molecular weight dextrin components (DP 8 to 19), and therefore exhibits low sweetness, low osmotic pressure, and moisture absorption resistance compared to general oligosaccharides, and also has a physical property modification effect due to the tendency of linear sugar molecules to crystallize. In addition, since the content of a DP of 20 or more is low, the dextrin-specific odor that may impair the aroma of foods and beverages, cosmetics, etc. is reduced. Therefore, it can be suitably applied to applications that do not require sweetness. For example, it can be used in food additives, foods and beverages, cosmetics, and medicines in which the use of oligosaccharides with high sweetness is undesirable. In addition, it can be used in foods and beverages, cosmetics, etc. in which the use of dextrin was difficult due to the strong odor specific to dextrin, without impairing the odor of the foods and beverages, cosmetics, etc. In the present technique, the content of a DP of 8 to 19 and a DP of 20 or more in the starch decomposition products is a value measured by the method described in the examples below.

In the starch decomposition product used in the present technique, the content of a DP of 8 to 19 is not particularly limited as long as it is 32% or more, but it is preferably 40% or more, more preferably 45% or more, and even more preferably 50% or more. The reason for this is that as the content of a DP of 8 to 19 increases, it exhibits lower viscosity, lower sweetness, lower osmotic pressure, and lower hygroscopicity, and the effect of improving physical properties due to the tendency of linear sugar molecules to crystallize increases.

In addition, in the starch decomposition product used in the present technique, the content of a DP of 20 or more is not particularly limited as long as it is 30% or less, but it is preferably 28% or less, more preferably 26% or less, and even more preferably 25% or less. The reason for this is that the lower the content of a DP of 20 or higher, the more the characteristic odor of dextrin is reduced. In addition, the reason is that the solubility in water is improved, and thus, the use feeling when used in cosmetics etc. is improved.

Further, the lower limit of the content of a DP of 20 or more in the starch decomposition product according to the present technique is not particularly limited as long as it does not impair the effects of the present technique, but is preferably 15% or more, more preferably 18% or more.

The starch decomposition product used in the present technique preferably has an iodine color value of 0.35 or more, more preferably 0.40 or more. By using a starch decomposition product whose iodine color value is 0.35 or more, good hardness and plasticity can be imparted to the oil-in-water type emulsion composition. That is, by using a starch decomposition product having an iodine color value of 0.35 or more, the shape retention of the oil-in-water type emulsion composition can be maintained and the desired physical properties can be more reliably exhibited. Further, the iodine color value is preferably 2 or less, more preferably 1.5 or less, and even more preferably 1.1 or less. By setting the upper limit of the iodine color value within this range, the desired physical properties can be more reliably exhibited, and turbidity is less likely to occur in the production process of the starch decomposition product, making production easier.

In the present technique, the iodine color value of a starch decomposition product is a value measured by the following iodine color value measurement method.

(Method for measuring iodine color value)

To a test tube into which 5 mL of water had been dispensed, 25 mg of a sample (starch decomposition product) was added as solid content and mixed, and 100 $\mu$L of an iodine color solution (0.2 mass/volume % iodine and 2 mass/volume % potassium iodide) was added, and after stirring, the mixture was allowed to stand at 30°C for 20 minutes, and then the absorbance at 660 nm was measured using a spectrophotometer with a glass cell having an optical path length of 10 mm, and the difference from the absorbance measured when no sample was added was taken as the iodine color value.

The color reaction with iodine indicates the presence of a linear sugar chain with a DP of 16 or more, and in starch decomposition products with a high content of a DP of 20 or more, a color reaction occurs because many linear sugar chains with a DP of 16 or more are present, but in starch decomposition products with a low content of a DP of 20 or more, a color reaction does not usually occur, or even if it does, the iodine color value is very low. However, the starch decomposition product used in the present technique shows a color reaction with iodine, because the main components are a DP of 8 to 19, which is near the lower limit of the iodine color reaction, and there are many linear components, despite the low content of a DP of 20 or more. That is, in starch decomposition products with a low content of a DP of 20 or more, the iodine color value is an index showing the degree of the content of linear components.

The content of a DP of 8 or more in the starch decomposition product according to the present technique is not particularly limited as long as it does not impair the effects of the present technique, but is preferably 50% or higher, more preferably 60% or higher, and even more preferably 70% or higher. By using a starch decomposition product with a high content of a DP of 8 or more, it exhibits lower sweetness, lower osmotic pressure, and lower hygroscopicity, and also has a propertyimproving effect due to the tendency of linear sugar molecules to crystallize. In the present technique, the content of a DP of 8 or more in the starch decomposition product is a value measured by the method described in the examples below.

The residual rate of the starch decomposition product according to the present technique in a $\beta$-amylase digestion test is not particularly limited as long as it does not impair the effects of the present technique, but is preferably 20% or less, more preferably 15% or less. By using a starch decomposition product with a low residual rate in a $\beta$-amylase digestion test, i.e., a starch decomposition product containing a large amount of linear sugar molecules (details will be described later), the shape retention of the oil-in-water type emulsion composition can be maintained and the desired physical properties can be more reliably exhibited.

In the present technique, the residual rate in the β-amylase digestion test is a value measured by the method described in the examples below. Note that β-amylase is an enzyme that decomposes glucose polymers into maltose units from the non-reducing end, and it is known that the presence of branched bonds such as α-1,6 bonds stops the decomposition. Therefore, evaluation of starch decomposition products by a β-amylase digestion test serves as an index for the extent to which α-1,4 bonds have continuous linear parts from a structural perspective. That is, in the color reaction with iodine, it is an index for linear sugar molecules with a DP of 16 or more, and in the evaluation by the β-amylase digestion test, it is an index for linear sugar molecules in the entire starch decomposition product.

The DE (dextrose equivalent) of the starch decomposition product according to the present technique is not particularly limited as long as it does not impair the effects of the present technique, but DE is preferably 30 or less, more preferably 10 to 25, and even more preferably 13 to 20. By using a starch decomposition product having a DE within this range, it is possible to exhibit low sweetness, low osmotic pressure, and low hygroscopicity, and to impart good physical properties to the oil-in-water type emulsion composition.

Note that "DE (dextrose equivalent)" is also referred to as dextrose equivalent, and is a value in which reducing sugar is measured as glucose and which indicates its proportion to the total solid content (see, mathematical formula (1) below). This DE value is an index indicating the degree of hydrolysis (degree of decomposition) of starch, that is, the degree of progress of saccharification. Note that in the present technique, DE is a value measured by the method described in examples described later.

[Mathematical Formula 1]

$$DE = [(\text{direct reducing sugar (expressed as glucose)})/\text{total solid content}] \times 100 \tag{1}$$

The content of the starch decomposition product in the oil-in-water type emulsion composition according to the present technique is not particularly limited as long as it does not impair the effects of the present technique, but in the present technique, the lower limit thereof is preferably 1.5% by mass or more, more preferably 2.0% by mass or more, and still more preferably 2.5% by mass or more. By setting the content of the starch decomposition product in the oil-in-water type emulsion composition to 1.5% by mass or more, the emulsified state of the oil-in-water type emulsion composition can be more stabilized and appropriate fluidity can be imparted. The upper limit thereof is preferably 30% by mass or less, more preferably 25% by mass or less, and still more preferably 20% by mass or less. By controlling the content of the starch decomposition product in the oil-in-water type emulsion composition to 30% by mass or less, the fluidity of the oil-in-water type emulsion composition can be improved.

(4) Method for producing starch decomposition product

The method for obtaining the starch decomposition product used in the present technique is not particularly limited as long as it does not impair the effects of the present technique. For example, starch raw materials can be subjected to predetermined operations such as treatment using a common acid or enzyme, various chromatography, membrane separation, ethanol precipitation, and the like, in appropriate combination, to obtain the starch decomposition product.

One method for efficiently obtaining the starch decomposition product for use in this technique is to act at least a debranching enzyme and a branching enzyme on starch or starch decomposition intermediates. Debranching enzymes are the general term for enzymes that catalyze the hydrolysis of α-1,6-glucosidic bonds, which are the branching points of starch. Branching enzymes are the general term for enzymes that act on linear glucans linked by α-1,4-glucosidic bonds, to create α-1,6-glucosidic bonds.

That is, debranching enzymes are enzymes involved in decomposing branched chains of starch, and branching enzymes are enzymes used to synthesize branched chains of starch. Therefore, the two are usually not used together. However, by using a combination of both enzymes that have completely opposite effects, it is possible to reliably produce the starch decomposition product used in this technique. In this case, as for the order of action of both enzymes, it is preferable to have the debranching enzyme act simultaneously or after the branching enzyme acts.

The debranching enzyme is not particularly limited. For example, pullulanase (pullulan 6-glucan hydrolase) and amylo-1,6-glucosidase/4-α-glucanotransferase can be mentioned, and more preferred as an example, isoamylase (glycogen 6-glucanohydrolase) can be used.

Furthermore, the branching enzyme is also not particularly limited. For example, enzymes purified from animals, bacteria, etc., those purified from plants such as potatoes, rice seeds, corn seeds, etc., and commercially available enzyme preparations can be used.

In the method for producing a starch decomposition product used in the present technique, it is also possible to perform a step of removing impurities after the enzyme reaction. The method for removing impurities is not particularly limited, and one or more known methods can be used in combination. For example, methods such as filtration, activated carbon decolorization, ion purification, etc. can be mentioned.

Furthermore, the starch decomposition product used in this technique can be used as a liquid product containing the starch decomposition product after an enzyme reaction, but it can also be dehydration-dried and powdered by vacuum drying, spray drying, freeze drying, etc. It is also possible to fractionate and use some components by chromatography or membrane separation.

(5) Oily component

As for the types of oily components used in the oil-in-water type emulsion composition according to the present technique, oily components that can be used in general oil-in-water type emulsion compositions can be used freely in combination of one or more, as long as the effects of the present technique are not impaired. Examples of the oily components include oils and fats, processed oils and fats, waxes, higher alcohols, higher fatty acids, esters, hydrocarbons, silicones, and the like. For example, oils and fats such as soybean oil, rapeseed oil (including canola oil), corn oil, sunflower oil, safflower oil, cottonseed oil, sesame oil, perilla oil, linseed oil, peanut oil, olive oil, grapeseed oil, macadamia oil, hazelnut oil, oat oil, pumpkin seed oil, walnut oil, camellia oil, tea seed oil, perilla oil, borage oil, rice bran oil, wheat germ oil, jojoba oil, almond oil, evening primrose oil, castor oil, palm oil, palm olein, palm kernel oil, palm kernel olein, coconut oil, cacao fat, beef tallow, lard, chicken fat, milk fat, fish oil, seal fat, mink oil, squalene, algae oil, etc.; processed oils and fats obtained by subjecting oils and fats to processing such as hydrogenation, transesterification, fractionation, etc.; hydrocarbons such as synthetic triglycerides, squalane, liquid paraffin, petrolatum, ceresin, microcrystalline wax, isoparaffin, etc.; waxes such as carnauba wax, paraffin wax, spermaceti, beeswax, candelilla wax, lanolin, etc.; higher alcohols such as cetanol, stearyl alcohol, lauryl alcohol, cetostearyl alcohol, oleyl alcohol, behenyl alcohol, lanolin alcohol, hydrogenated lanolin alcohol, hexyldecanol, octyldodecanol, etc.; higher fatty acids such as lauric acid, myristic acid, palmitic acid, stearic acid, behenic acid, isostearic acid, oleic acid, linolenic acid, linoleic acid, oxystearic acid, undecylenic acid, lanolin fatty acid, hard lanolin fatty acid, soft lanolin fatty acid, etc.; cholesterol and its derivatives such as cholesteryl-octyldodecyl-behenyl, etc.; esters such as isopropyl myristic acid, isopropyl palmitic acid, isopropyl stearic acid, glycerol 2-ethylhexanoate, butyl stearic acid, etc.; polar oils such as diethylene glycol monopropyl ether, polyoxyethylene polyoxypropylene pentaerythritol ether, polyoxypropylene butyl ether, ethyl linoleate, etc.; others: amino-modified silicone, epoxy-modified silicone, carboxyl-modified silicone, carbinol-modified silicone, methacrylic-modified silicone, mercapto-modified silicone, phenol-modified silicone, single-end reactive silicone, heterofunctional group-modified silicone, polyether-modified silicone, methylstyrylmodified silicone, alkyl-modified silicone, higher fatty acid ester-modified silicone, hydrophilic special modified silicone, higher alkoxy-modified silicone, higher fatty acid-containing silicone, fluorine-modified silicone, etc., and more specifically, silicone resins, and silicones containing various derivatives such as methylphenylpolysiloxane, methylpolysiloxane, octa methylcyclotetrasiloxane, decamethylcyclopentasiloxane, dodecamethylcyclohexanesiloxane, methylcyclopolysiloxane, octamethyltrisiloxane, decamethyltetrasiloxane, polyoxyethylene/methylpolysiloxane copolymer, polyoxypropylene/methylpolysiloxane copolymer, poly(oxyethylene/oxypropylene) methylpolysiloxane copolymer, methylhydrogenpolysiloxane, tetrahydrotetramethylcyclotetrasiloxane, stearoxymethylpolysiloxane, cetoxymethylpolysiloxane, methylpolysiloxane emulsion, high polymerization methylpolysiloxane, trimethylsiloxysilicic acid, crosslinked methylpolysiloxane, crosslinked methylphenylpolysiloxane, crosslinked methylphenylpolysiloxane, etc. can be used alone or in combination. Note that oily components derived from plants may be made from plants that have been improved in varieties using genetic recombination technology as raw materials, and for rapeseed oil, sunflower oil, safflower oil, soybean oil, etc., for example, high oleic acid type varieties having an enhanced oleic acid content can be used.

The content of the oily component in the oil-in-water type emulsion composition according to the present technique is not particularly limited as long as it does not impair the effects of the present technique, but in the present technique, the lower limit thereof is preferably 15% by mass or more, more preferably 20% by mass or more, and even more preferably 25% by mass or more. By setting the content of the oily component in the oil-in-water type emulsion composition to 15% by mass or more, the food texture of the oil-in-water type emulsion composition can be made smooth. The upper limit thereof is preferably 65% by mass or less, more preferably 60% by mass or less, and even more preferably 55% by mass or less. By controlling the content of the oily component in the oil-in-water type emulsion composition to 65% by mass or less, the oil-in-water type emulsion composition can have a reduced fatty feel and can suppress separation.

(6) Emulsifier

The oil-in-water type emulsion composition according to the present technique may further contain an emulsifier. Although an emulsifier is not an essential component in the present technique, by including an emulsifier in the oil-in-water type emulsion composition according to the present technique, the emulsion structure is strengthened, and the stability of the oil-in-water type emulsion composition can be further improved.

As the emulsifier that can be used in the present technique, depending on the purpose of the oil-in-water type emulsion composition, one or more emulsifiers generally used for the purpose can be freely selected and used, as long as the effect of the present technique is not impaired. Examples thereof include polyoxyethylene alkyl ether, polyoxyethylene alkylphenyl ether, polyoxyethylene steryl ether, polyoxyethylene polyoxypropylene alkyl ether, polyoxyethylene fatty acid ester, polyoxyethylene polyhydric alcohol fatty acid ester, polyoxyethylene hydrogenated castor oil, polyoxyethylene sorbitan fatty acid ester, glycol fatty acid ester, glycerin fatty acid ester, sorbitan fatty acid ester, propylene glycol fatty acid ester, sucrose fatty acid ester, polyglycerin fatty acid ester, fatty acid alkanolamide, alkyl glycoside, saponin, lecithin, and cyclic oligosaccharides such as $\alpha$-cyclodextrin, etc.

The content of the emulsifier in the oil-in-water type emulsion composition according to the present technique is not particularly limited as long as it does not impair the effects of the present technique, but in the present technique, the lower limit thereof is preferably 0.5% by mass or more, more preferably is 0.7% by mass or more, and even more preferably 1.0% by mass or more. By setting the content of the emulsifier in the oil-in-water type emulsion composition to 0.5% by mass or more, the shape retention of the oil-in-water type emulsion composition can be improved. The upper limit thereof is preferably 5% by mass or less, more preferably 4% by mass or less, and even more preferably 3% by mass or less. By controlling the content of the emulsifier in the oil-in-water type emulsion composition to 5% by mass or less, the fluidity of the oil-in-water type emulsion composition can be improved.

In this technique, it is preferable to use a cyclic oligosaccharide as the emulsifier. By containing a cyclic oligosaccharide in the oil-in-water type emulsion composition according to the present technique, the emulsion structure becomes even stronger, and the stability of the oil-in-water type emulsion composition can be further improved. Cyclic oligosaccharides are non-synthetic emulsifiers, but as shown in the examples below, with this technique, oil-in-water type emulsion compositions with good physical properties can be produced by using cyclic oligosaccharides even without using synthetic emulsifiers.

The type of the cyclic oligosaccharide that can be used in the present technique is not particularly limited as long as it does not impair the effects of the present technique, and any of the three types of cyclodextrin ($\alpha$, $\beta$, $\gamma$) commercially available can be used. However, in consideration of the interaction with oily components, it is desirable to select $\alpha$-cyclodextrin in the present technique.

(7) Thickener

The oil-in-water type emulsion composition according to the present technique may further contain a thickener. Although a thickener is not an essential component in the present technique, the inclusion of a thickener in the oil-in-water type emulsion composition according to the present technique can facilitate viscosity control or further increase the viscosity of the oil-in-water type emulsion composition.

The type of the thickener that can be used in the present technique is not particularly limited as long as it does not impair the effects of the present technique, and depending on the purpose of the oil-in-water type emulsion composition, one or more thickeners generally used for the purpose can be freely selected and used. Examples thereof include gums such as xanthan gum, tamarind seed gum, gellan gum, gum arabic, guar gum, tara gum, tremel gum, etc.; and hydroxypropylmethylcellulose (HPMC), carboxymethylcellulose (CMC), sodium alginate, carboxyvinyl polymer (carbomer), sodium polyacrylate, (ethylene/propylene) copolymer, and the like.

The content of the thickener when used in the present technique is not particularly limited as long as it does not impair the effects of the present technique. The lower limit of the content of the thickener in the oil-in-water type emulsion composition according to the present technique is, for example, 0.05% by mass or more, preferably 0.1% by mass or more. The upper limit of the content of the thickener in the oil-in-water type emulsion composition according to the present technique is, for example, 3% by mass or less, preferably 1% by mass or less.

(8) Other components

In the oil-in-water type emulsion composition according to the present invention, one or more other components that can be used in general oil-in-water type emulsion compositions can be freely selected and contained, as long as the effects of the present technique are not impaired. As other components, components such as, for example, excipients, pH adjusters, colorants, fragrances, tasting agents, flavoring agents, disintegrants, lubricants, stabilizers, moisturizers, preservatives, and the like, can be used. Specific examples thereof include amino acids such as arginine, etc.; glycerin, propylene glycol, PEG-8, paraben, and the like.

Furthermore, components having known or future-discovered functions may be used in combination depending on the purpose. The starch decomposition products mentioned above are classified as foods and are derived from plants, so depending on the selection of components other than the starch decomposition products, it is also possible to handle foods and cosmetics containing the starch decomposition products, the oil-in-water type emulsion composition according to the present invention as a product without the addition of synthetic additives or as a product derived from plants.

2. Cosmetic

The oil-in-water type emulsion composition according to the present technique described above can be suitably used in cosmetics. Cosmetics that can use the oil-in-water type emulsion composition according to the present technique are not particularly limited, and examples thereof include skin care cosmetics such as lotion, milky lotion, cream, eye cream, beauty essence, massage agent, pack agent, hand cream, body cream, cleansing agent, detergent, sunscreen agent, etc.; makeup cosmetics such as powder foundation, liquid foundation, cream foundation, makeup base, blemish balm cream (BB cream), control color cream (CC cream), etc.; and hair cosmetics such as shampoo, hair rinse, hair pack, hair treatment, hair conditioner, hair lotion, split end coat agent, hair milk, hair cream, etc.

3. Food

The oil-in-water type emulsion composition according to the present technique described above can also be suitably used for foods. Foods to which the oil-in-water type emulsion composition according to the present technique can be used are not particularly limited, and examples thereof include seasonings such as mayonnaise, ketchup, sauces, etc.; creams such as custard cream, etc.; various dairy products such as flower pastes, soups, yogurt, etc.; frozen desserts such as ice cream, etc.; and processed foods such as preserved foods, frozen foods, breads, sweets, cooked rice, noodles, water paste products, meat products, etc. In addition, this technique can also be used in foods and beverages with health functions (including foods for specified health function, foods with functional claims, and foods with nutritional functions), so-called health foods (including beverages), liquid foods, infant and toddler foods, diet foods, diabetic foods, and the like.

EXAMPLES

The present technique will be described in more detail below based on examples. Note that the examples described below are representative examples of the present technique, and the scope of the present technique is not to be interpreted narrowly by these examples.

(1) Test method

[Branching enzyme]

In this experimental example, a potato-derived enzyme (hereinafter, referred to as "potato-derived branching enzyme") purified in accordance with the method of Eur. J. Biochem. 59, p615-625 (1975) and Branchzyme (manufactured by Novozymes Inc., hereinafter, referred to as "bacterium-derived branching enzyme") were used, as examples of branching enzymes.
The activity of the branching enzyme was measured as follows.
As a substrate solution, an amylose solution was used in which 0.1% by mass of amylose (Sigma-Aldrich, A0512) was dissolved in 0.1 M acetate buffer (pH 5.2). Fifty microliters (50 μL) of an enzyme solution was added to 50 μL of the substrate solution, and the mixture was reacted at 30°C for 30 minutes, after which 2 mL of an iodine-potassium iodide solution (0.39 mM iodine-6 mM potassium iodide-3.8 mM hydrochloric acid mixture) was added to stop the reaction. A blank solution was prepared by adding water instead of the enzyme solution. The absorbance at 660 nm was measured 15 minutes after the reaction was stopped. One unit of enzyme activity of the branching enzyme was defined as the enzyme activity that reduces the absorbance at 660 nm by 1% per minute when tested under the above conditions.

[Content of a DP of 8 to 19, a DP of 20 or more, and a DP of 8 or more]

Analysis was performed by high performance liquid chromatography (HPLC) under the conditions shown in Table 1 below, and the contents of a DP of 8 to 19, a DP of 20 or more, and a DP of 8 or more were measured based on the detected peak area ratios.

[Table 1]

| Column | MCI CK02AS (manufactured by Mitsubishi Chemical Corporation) |
|---|---|
| Column temperature | 80°C |

(continued)

| Eluent | Water |
| --- | --- |
| Flow rate | 1.0 mL/min |
| Detector | Differential refractometer |

[Measurement of iodine color value]

Twenty five milligrams (25 mg) of a sample (starch decomposition product) was added as a solid content to a test tube into which 5 mL of water had been dispensed, and mixed. To this, 100 μL of an iodine color solution (0.2 mass/volume % iodine and 2 mass/volume % potassium iodide) was added, and after stirring, the mixture was allowed to stand at 30°C for 20 minutes, and then the absorbance at 660 nm was measured using a spectrophotometer with a glass cell having an optical path length of 10 mm, and the difference from the absorbance measured when no sample was added was taken as the iodine color value.

[Residual rate in β-amylase digestion test]

A starch decomposition product was dissolved in 10 mM acetate buffer (pH 5.5) by boiling to prepare 10 mL of a solution with a solid concentration of 10% by mass, and to this was added 10 μL of β-amylase (Nagase ChemteX Corporation), and the reaction was allowed to proceed at 55°C for 72 hours, after which the reaction was terminated by heat treatment at 100°C for 10 minutes. The reaction solution was desalted with an ion exchange resin, and the content of a DP of 4 or more was measured by the following method, and the value was taken as the residual rate.

[Table 2]

| Column | MCI CK04S (manufactured by Mitsubishi Chemical Corporation) |
| --- | --- |
| Column temperature | 65°C |
| Eluent | Water |
| Fow rate | 0.35 mL/min |
| Detector | Differential refractometer |

[DE]

The DE was calculated according to the Lane-Eynon method described on pages 5-6 of "Starch and Sugar Related Industry Analysis Method" (ed., Starch and Sugar Technology Committee).

(2) Production of starch decomposition product

[Starch decomposition product 1]

To a 30 % by mass corn starch slurry adjusted to pH 5.8 with 10 % by mass calcium hydroxide, 0.2 % by mass α-amylase (Liquozyme Supra, manufactured by Novozymes Japan Ltd.) was added per solid content (g), and it was liquefied in a jet cooker (temperature 110°C). This liquefied liquid was kept at 95°C, DE was measured over time, and when DE reached 8, the pH was adjusted to 4 with 10% by mass hydrochloric acid, and the reaction was stopped by boiling. After the reaction was stopped, the pH of the sugar solution was adjusted to 5.8, and then 2000 units of a potato-derived branching enzyme was added per solid content (g), and the mixture was reacted at 35°C for 24 hours. Thereafter, 1.5% by mass of a debranching enzyme (GODO-FIA, manufactured by Godo Shusei Co., Ltd.) was added per solid content (g), and the mixture was reacted at 50°C for 24 hours. This starch decomposition product solution was decolorized with activated carbon, ion-purified, and concentrated to a solid content concentration of 40% by mass. The concentrated liquid was pulverized using a spray dryer to obtain a starch decomposition product 1.

[Starch decomposition product 2]

To a 30 % by mass corn starch slurry adjusted to pH 5.8 with 10 % by mass calcium hydroxide, 0.2 % by mass α-amylase (Liquozyme Supra, manufactured by Novozymes Japan Ltd.) was added per solid content (g), and it was liquefied in a jet cooker (temperature 110°C). This liquefied liquid was kept at 95°C, DE was measured over time, and when DE reached 8, the pH was adjusted to 4 with 10% by mass hydrochloric acid, and the reaction was stopped by boiling. After the reaction was stopped, the pH of the sugar solution was adjusted to 5.8, and then 500 units of a bacterium-derived branching enzyme was added per solid content (g), and the mixture was reacted at 65°C for 40 hours. Thereafter, 0.5% by mass of a debranching enzyme (GODO-FIA, manufactured by Godo Shusei Co., Ltd.) was added per solid content (g), and the mixture was reacted at 50°C for 48 hours. This starch decomposition product solution was decolorized with activated carbon, ion-purified, and concentrated to a solid content concentration of 40% by mass. The concentrated liquid was pulverized using a spray dryer to obtain a starch decomposition product 2.

[Starch decomposition product 3]

To a 30% by mass corn starch slurry adjusted to pH 5.8 with 10% by mass calcium hydroxide, 0.2% by mass of $\alpha$-amylase (Kleistase T10S, manufactured by Amano Enzyme Inc.) was added per solid content (g), and it was liquefied in a jet cooker (temperature 110°C). The liquefied liquid was kept at 95°C, DE was measured over time, and when the DE reached 9, the pH was adjusted to 4 with 10% by mass hydrochloric acid, and the reaction was stopped by boiling. After the reaction was stopped, the pH of the sugar solution was adjusted to 5.8, and then 800 units of a bacterium-derived branching enzyme was added per solid content (g), and the mixture was reacted at 65°C for 30 hours. Thereafter, 1.0% by mass of a debranching enzyme (GODO-FIA, manufactured by Godo Shusei Co., Ltd.) was added per solid content (g), and the mixture was reacted at 50°C for 30 hours. The starch decomposition product solution was decolorized with activated carbon, ion-purified, and concentrated to a solid content concentration of 50% by mass. The concentrated liquid was pulverized using a spray dryer to obtain a starch decomposition product 3.

[Starch decomposition product 4]

To a 30% by mass cornstarch slurry adjusted to pH 5.8 with 10% by mass calcium hydroxide, 0.2% by mass of $\alpha$-amylase (Kleistase T10S, manufactured by Amano Enzyme Inc.) was added per solid content (g), and it was liquefied in a jet cooker (temperature 110°C). This liquefied liquid was kept at 95°C, DE was measured over time, and when DE reached 8, the pH was adjusted to 4 with 10% by mass hydrochloric acid, and the reaction was stopped by boiling. After the reaction was stopped, the pH of the sugar solution was adjusted to 5.8, and then 600 units of a bacterium-derived branching enzyme was added per solid content (g), and the mixture was reacted at 65°C for 15 hours. Thereafter, 0.5% by mass of a debranching enzyme (GODO-FIA, manufactured by Godo Shusei Co., Ltd.) was added per solid content (g), and the mixture was reacted at 50°C for 40 hours. This starch decomposition product solution was decolorized with activated carbon, ion-purified, and concentrated to a solid content concentration of 45% by mass. The concentrated liquid was pulverized using a spray dryer to obtain a starch decomposition product 4.

[Starch decomposition product 5]

A 30% by mass cornstarch slurry adjusted to pH 2 with 10% by mass hydrochloric acid was decomposed to DE13 at a temperature of 130°C. After returning to normal pressure, the reaction was stopped by neutralizing with 10% by mass sodium hydroxide, and the pH of the sugar solution was adjusted to 5.8, and then 400 units of a bacterium-derived branching enzyme was added per solid content (g), and the mixture was reacted at 65°C for 48 hours. Thereafter, 1.0% by mass of a debranching enzyme (GODO-FIA, manufactured by Godo Shusei Co., Ltd.) was added per solid content (g), and the mixture was reacted at 50°C for 60 hours. This starch decomposition product solution was decolorized with activated carbon, ion-purified, and pulverized using a spray dryer to obtain a starch decomposition product 5.

[Starch decomposition product 6]

A 30% by mass waxy cornstarch slurry adjusted to pH 2 with 10% by mass hydrochloric acid was decomposed to DE6 at a temperature of 130°C. After returning to normal pressure, the reaction was stopped by neutralizing with 10% by mass sodium hydroxide, and the pH of the sugar solution was adjusted to 5.8, and then 500 units of a bacterium-derived branching enzyme was added per solid content (g), and 0.5% by mass of a debranching enzyme (GODO-FIA, manufactured by Godo Shusei Co., Ltd.) was added at per solid content (g), and the mixture was reacted at 50°C for 72 hours. This starch decomposition product solution was decolorized with activated carbon, ion-purified, and concentrated to a solid content concentration of 40% by mass. The concentrated liquid was pulverized using a spray dryer to obtain a starch decomposition product 6.

[Starch decomposition product 7]

To a 30 % by mass corn starch slurry adjusted to pH 5.8 with 10 % by mass calcium hydroxide, 0.2 % by mass α-amylase (Liquozyme Supra, manufactured by Novozymes Japan Ltd.) was added per solid content (g), and it was liquefied in a jet cooker (temperature 110°C). This liquefied liquid was kept at 95°C, DE was measured over time, and when DE reached 15, the pH was adjusted to 4 with 10% by mass hydrochloric acid, and the reaction was stopped by boiling. This starch decomposition product solution was decolorized with activated carbon, ion-purified, and concentrated to a solid content concentration of 40% by mass. The concentrated liquid was pulverized using a spray dryer to obtain a starch decomposition product of Comparative Example 1.

(3) Measurement

For the starch decomposition products 1 to 7 obtained above, the contents of a DP of 8 to 19, a DP of 20 or more and a DP of 8 or more, the iodine color value, the residual rate in β-amylase digestion test, and DE were measured by the method described above. The results are shown in Table 3 below.

[Table 3]

| | | Saccharide composition (content%) | | | Iodine color value | Residual rate in β amylase digestion test | DE |
|---|---|---|---|---|---|---|---|
| | | DP 8 to 19 | DP 20 or more | DP 8 or more | | | |
| Starch decomposition product | 1 | 52 | 21 | 73 | 0.74 | 9 | 14 |
| | 2 | 54 | 23 | 77 | 0.49 | 10 | 15 |
| | 3 | 55 | 24 | 79 | 0.64 | 9 | 15 |
| | 4 | 49 | 29 | 78 | 1.28 | 11 | 16 |
| | 5 | 50 | 16 | 66 | 0.35 | 6 | 18 |
| | 6 | 52 | 24 | 76 | 1.05 | 8 | 16 |
| | 7 | 20 | 46 | 66 | 0.26 | 26 | 15 |

<Experimental Example 1>

In Experimental Example 1, when producing an oil-in-water type emulsion composition without using a synthetic emulsifier, it was investigated how the properties of starch decomposition products affect the physical properties of the oil-in-water type emulsion composition. In Experimental Example 1, a cosmetic was produced as an example of the oil-in-water type emulsion composition.

(1) Material

Starch decomposition product: See Table 3 above
Emulsifier: Cyclic oligosaccharide (a-cyclodextrin: "Dexypearl K-100" manufactured by Ensuiko Sugar Refining Co., Ltd.)
Thickener: Xanthan gum ("Echo Gum (registered trademark)" manufactured by Sumitomo Pharma Food & Chemical Co., Ltd.)
Oils and Fats (oily component): Edible rice oil ("Kenkou Kome Abura (Healthy Rice Oil)" manufactured by Showa Sangyo Co., Ltd.)

Preservative: Ethylparaben

(2) Production of cosmetic

Cosmetics were produced according to the formulations in Table 4 below, using the following procedures.

1. A starch decomposition product, an emulsifier, and a thickener were dissolved in water at 70°C.

2. The aqueous solution of 1, and oils and fats in which a preservative was dissolved were homogenized at 12,000 rpm for 3 minutes using a homogenizer.

3. It was transferred to a container and stored at room temperature for 2 days.

(3) Evaluation

The viscosity of the oil-in-water type emulsion composition of each sample was measured using the following method. In addition, a panel of 10 experts discussed and evaluated the fluidity of the oil-in-water type emulsion composition of each sample based on the tactile sensation when slowly pressing the oil-in-water type emulsion composition filled in a container with a finger, based on the evaluation criteria below. Assuming that the score of the fluidity of each oil-in-water type emulsion (samples 7, 9, 11, 13, 15, 17, 19, and 21) using the starch decomposition product 7 is 3 points (control), the fluidity of each oil-in-water type emulsion (samples 1 to 6, 8, 10, 12, 14, 16, 18, and 20) produced while replacing the starch decomposition product 7 with the starch decomposition product according to the present technique was evaluated, respectively.

[Viscosity]

A 25 mm parallel plate was set in a rheometer MCR102 (manufactured by Anton Paar), and the viscosity was measured at 25°C and a rotation speed of 200/s.

[Fluidity]

1 point: Very soft or very fluid compared to control
2 points: Softer or more fluid than control
3 points: Control
4 points: Hard or less fluid compared to control
5 points: Very hard or much less fluid compared to control

(4) Results

The results are shown in Table 4 below.

[Table 4]

| | | Sample | | | | | | |
|---|---|---|---|---|---|---|---|---|
| | | 1 | 2 | 3 | 4 | 5 | 6 | 7 |
| Composition (mass%) | Water | 33.6 | 33.6 | 33.6 | 33.6 | 33.6 | 33.6 | 33.6 |
| | Starch decomposition product 1 | 4.0 | - | - | - | - | - | - |
| | Starch decomposition product 2 | - | 4.0 | - | - | - | - | - |
| | Starch decomposition product 3 | - | - | 4.0 | - | - | - | - |
| | Starch decomposition product 4 | - | - | - | 4.0 | - | - | - |
| | Starch decomposition product 5 | - | - | - | - | 4.0 | - | - |
| | Starch decomposition product 6 | - | - | - | - | - | 4.0 | - |
| | Starch decomposition product 7 | - | - | - | - | - | - | 4.0 |
| | Emulsifier | 2.0 | 2.0 | 2.0 | 2.0 | 2.0 | 2.0 | 2.0 |
| | Thickener | 0.3 | 0.3 | 0.3 | 0.3 | 0.3 | 0.3 | 0.3 |
| | Oils and fats | 60.0 | 60.0 | 60.0 | 60.0 | 60.0 | 60.0 | 60.0 |
| | Preservative | 0.1 | 0.1 | 0.1 | 0.1 | 0.1 | 0.1 | 0.1 |
| | Total | 100.0 | 100.0 | 100.0 | 100.0 | 100.0 | 100.0 | 100.0 |
| | Starch decomposition product /moisture | 0.12 | 0.12 | 0.12 | 0.12 | 0.12 | 0.12 | 0.12 |
| Evaluation | Viscosity (Pa・s) | 2.1 | 2.0 | 2.1 | 2.1 | 2.2 | 1.9 | 3.3 |
| | Fluidity | 4.5 | 4.5 | 4.4 | 4.5 | 4.0 | 4.4 | 3.0 |

(5) Discussion

As shown in Table 4, samples 1 to 6 using starch decomposition products 1 to 6 which had a content of a glucose polymerization degree (DP) of 8 to 19 of 32% or more and a content of a glucose polymerization degree (DP) of 20 or higher of 30% or less and in which the starch decomposition product/water = 0.05 or more gave results in which hardness was good and the viscosity at a rotational speed of 200/s was low, as compared with a sample 7 using the same amount of the starch decomposition product 7. Due to these properties, cosmetics harden when stored, and their viscosity decreases when applied to the skin or hair, making it possible to achieve a high use feeling. Note that even when an experiment was conducted using 0.5% by mass of sucrose fatty acid ester (synthetic emulsifier) as an emulsifier instead of the cyclic oligosaccharide, good results were obtained with the product of the present invention.

<Experimental Example 2>

In Experimental Example 2, when producing an oil-in-water type emulsion composition without using a synthetic emulsifier, it was examined how various components and formulations affect the physical properties of the oil-in-water type emulsion composition. In Experimental Example 2, a cosmetic was produced as an example of the oil-in-water type emulsion composition.

(1) Material

Starch decomposition product: See Table 3 above
Emulsifier: Cyclic oligosaccharide ($\alpha$ cyclodextrin: "Dexypearl K-100" manufactured by Ensuiko Sugar Refining Co., Ltd.)
Thickener: Xanthan gum ("Echo Gum (registered trademark)" manufactured by Sumitomo Pharma Food & Chemical Co., Ltd.)

Oils and fats (oily component): Edible sunflower oil ("Olein Rich" manufactured by Showa Sangyo Co., Ltd.)

Preservative: Ethylparaben

(2) Production of cosmetic

Cosmetics were produced according to the formulations in Table 5 below and according to the following procedure.

1. A starch decomposition product, an emulsifier, and a thickener were dissolved in 70°C water.
2. The aqueous solution of 1, and oils and fats containing a preservative dissolved therein were homogenized using a homogenizer at 12,000 rpm for 3 minutes.
3. It was transferred to a container and stored at room temperature for 7 days.

(3) Evaluation

The viscosity of the oil-in-water type emulsion composition of each sample was measured using the same method as in Experimental Example 1 above. Furthermore, the fluidity of the oil-in-water type emulsion composition of each sample was evaluated based on the same evaluation criteria as in Experimental Example 1 above.

(4) Results

The results are shown in Table 5 below.

[Table 5]

| Composition (mass%) | Sample | | | | | | | | | | | | | | | |
| --- | 8 | 9 | 10 | 11 | 12 | 13 | 14 | 15 | 16 | 17 | 18 | 19 | 20 | 21 | 22 | 23 |
| Water | 36.6 | 36.6 | 35.6 | 35.6 | 47.6 | 47.6 | 52.6 | 52.6 | 29.6 | 29.6 | 28.9 | 28.9 | 27.6 | 27.6 | 22.6 | 22.6 |
| Starch decomposition product 1 | 1.0 | - | 2.0 | - | 20.0 | - | 25.0 | - | 18.0 | - | 18.0 | - | 20.0 | - | 25.0 | - |
| Starch decomposition product 7 | - | 1.0 | - | 2.0 | - | 20.0 | - | 25.0 | - | 18.0 | - | 18.0 | - | 20.0 | - | 25.0 |
| Emulsifier | 2.0 | 2.0 | 2.0 | 2.0 | 2.0 | 2.0 | 2.0 | 2.0 | 2.0 | 2.0 | 3.0 | 3.0 | 2.0 | 2.0 | 2.0 | 2.0 |
| Thickener | 0.3 | 0.3 | 0.3 | 0.3 | 0.3 | 0.3 | 0.3 | 0.3 | 0.3 | 0.3 | - | - | 0.3 | 0.3 | 0.3 | 0.3 |
| Oils and fats | 60.0 | 60.0 | 60.0 | 60.0 | 30.0 | 30.0 | 20.0 | 20.0 | 50.0 | 50.0 | 50.0 | 50.0 | 50.0 | 50.0 | 50.0 | 50.0 |
| Preservative | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Total | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| **Evaluation** | | | | | | | | | | | | | | | | |
| Starch decomposition product/moisture | 0.03 | 0.03 | 0.06 | 0.06 | 0.42 | 0.42 | 0.48 | 0.48 | 0.61 | 0.61 | 0.62 | 0.62 | 0.72 | 0.72 | 1.11 | 1.11 |
| Viscosity (Pa·s) | 2.4 | 1.7 | 1.8 | 2.3 | 0.5 | 0.6 | 0.2 | 0.3 | 1.6 | 2.9 | 1.7 | 2.6 | 1.1 | 4.1 | *1 | *2 |
| Fluidity | 3.0 | 3.0 | 3.5 | 3.0 | 4.6 | 3.0 | 4.4 | 3.0 | 4.8 | 3.0 | 4.7 | 3.0 | 4.9 | 3.0 | - | *2 |

*1: Not measurable. (No oil separation etc.)
*2: It could not be evaluated because the oil had separated.

(5) Discussion

As shown in Table 5, samples 10, 12, 14, 16, 18, and 20 using the starch decomposition product 1 which had a content of a glucose polymerization degree (DP) of 8 to 19 of 32% or more and a content of a glucose polymerization degree (DP) of 20 or more of 30% or less and in which the starch decomposition product/water = 0.05 or more gave results in which hardness was good and the viscosity at a rotational speed of 200/s was low, as compared with samples 11, 13, 15, 17, 19, and 21 using the same amount of the starch decomposition product 7. The sample 22 hardened strongly and the plate did not lower to a prescribed position, and therefore, the viscosity thereof could not be measured, but it was an emulsified composition with good spread texture. In the sample 23, oil and fats separated, and an emulsified composition could not be produced. Due to these properties, when storing cosmetics, they remain solid or viscous, and when applied to the skin or hair, the viscosity decreases, making it possible to achieve a high use feeling. On the other hand, the sample 9 using the starch decomposition product 1 in which the content of a glucose polymerization degree (DP) of 8 to 19 was 32% or more and the content of a glucose polymerization degree (DP) of 20 or more was 30% or less and in which the starch decomposition product/water = 0.03 gave results in which fluidity had no difference and the viscosity at a rotation speed of 200/s was high, compared with the sample 10 using the same amount of the starch decomposition product 7.

For samples 1, 10, 12, 14, 16, 18 and 20, a 25 mm parallel plate was set in a rheometer MCR102 (manufactured by Anton Paar Inc.) and the viscosity was measured at 25°C and a rotation speed of 2/s, resulting in the viscosity values shown in Table 6. Table 6 also shows the viscosity at a rotation speed of 200/s and the difference between the viscosity at a rotation speed of 2/s and the viscosity at a rotation speed of 200/s.

[Table 6]

|  |  |  | Sample | | | | | | |
|---|---|---|---|---|---|---|---|---|---|
|  |  |  | 1 | 10 | 12 | 14 | 16 | 18 | 20 |
| Viscosity (Pa · s) | Rotational speed (1/s) | 2 | 150.0 | 129.0 | 16.7 | 8.7 | 161.0 | 209.0 | 900.0 |
|  |  | 200 | 2.1 | 1.8 | 0.5 | 0.2 | 1.6 | 1.7 | 1.1 |
|  | Difference in viscosity between 2/s and 200/s | | 147.9 | 127.2 | 16.2 | 8.5 | 159.4 | 207.3 | 898.9 |

<Experimental Example 3>

A cosmetic cream was produced according to a conventional method using the formulation shown in Table 7. As a result, a good cosmetic cream was obtained.

[Table 7]

| Component | Blended amount (mass%) |
|---|---|
| Water | 30.8 |
| Starch decomposition product 1 | 5.0 |
| Cetostearyl alcohol* 1 | 4.0 |
| Squalane* 1 | 40.0 |
| Liquid paraffin* 1 | 5.0 |
| Beeswax* 1 | 3.0 |
| Reduced lanolin* 1 | 5.0 |
| Monostearic acid glyceride*2 | 2.0 |
| Glycerin | 5.0 |
| Methylparaben | 0.1 |
| Ethylparaben | 0.1 |
| Total | 100.0 |
| *1: Oily component<br>*2: Emulsifier | |

<Experimental Example 4>

A cosmetic milky lotion was produced according to a conventional method using the formulation shown in Table 8. As a result, a good cosmetic milky lotion was obtained.

[Table 8]

| Component | Blended amount (mass%) |
|---|---|
| Water | 68.6 |
| Starch decomposition product 1 | 5.0 |
| Stearic acid*1 | 2.5 |
| Cetyl alcohol*1 | 1.5 |
| Vaseline*1 | 5.0 |
| Liquid paraffin* 1 | 10.0 |
| Polyoxyethylene sorbitan monooleate*2 | 2.0 |
| Carboxyl vinyl polymer*3 | 0.2 |
| Propylene glycol | 5.0 |
| Methylparaben | 0.1 |
| Ethylparaben | 0.1 |
| Total | 100.0 |
| *1: Oily component<br>*2: Emulsifier<br>*3: Thickener | |

<Experimental Example 5>

A conditioner was produced according to a conventional method using the formulation shown in Table 9. As a result, a good conditioner was obtained.

[Table 9]

| Component | Blended amount (mass%) |
|---|---|
| Water | 69.6 |
| Starch decomposition product 1 | 5.0 |
| Cetyl alcohol*1 | 5.0 |
| Oleyl alcohol*1 | 1.0 |
| Olive oil*1 | 2.0 |
| Vaseline*1 | 8.0 |
| Behentrimonium chloride | 2.0 |
| Stearic acid glyceride*2 | 1.0 |
| Glycerin | 6.0 |
| Arginine | 0.2 |
| Methylparaben | 0.1 |
| Ethylparaben | 0.1 |
| Total | 100 |
| *1: Oily component<br>*2: Emulsifier | |

<Experimental Example 6>

A facial cleanser was produced according to a conventional method using the formulation shown in Table 10. As a result, a good facial cleanser was obtained.

[Table 10]

| Component | Blended amount (mass%) |
| --- | --- |
| Water | 38.6 |
| Starch decomposition product 1 | 3.0 |
| Lauric acid*1 | 10.0 |
| Palmitic acid*1 | 10.0 |
| Stearic acid*1 | 10.0 |
| Stearic acid glyceride*2 | 2.0 |
| Glycerin | 10.0 |
| PEG-8 | 10.0 |
| K Hydroxide | 6.0 |
| Arginine | 0.2 |
| Methylparaben | 0.1 |
| Ethylparaben | 0.1 |
| Total | 100 |
| *1: Oily component<br>*2: Emulsifier | |

## Claims

1. An oil-in-water type emulsion composition comprising:

   a starch decomposition product having
   a content of a glucose polymerization degree (DP) of 8 to 19 of 32% or more and
   a content of a glucose polymerization degree (DP) of 20 or more of 30% or less;
   water; and
   an oily component;
   wherein the ratio of the starch decomposition product/water is 0.05 or more.

2. The oil-in-water type emulsion composition according to claim 1, which is for use in cosmetics.

3. The oil-in-water type emulsion composition according to claim 1, wherein the starch decomposition product has an iodine color value of 0.35 or more.

4. The oil-in-water type emulsion composition according to claim 1, comprising 1.5 to 30% by mass of the starch decomposition product.

5. The oil-in-water type emulsion composition according to claim 1, comprising 15 to 65% by mass of the oily component.

6. The oil-in-water type emulsion composition according to claim 1, which comprises an emulsifier.

7. The oil-in-water type emulsion composition according to claim 6, comprising 0.5 to 5% by mass of the emulsifier.

8. The oil-in-water type emulsion composition according to claim 6, wherein the emulsifier contains a cyclic oligosaccharide.

9. The oil-in-water type emulsion composition according to claim 8, wherein the cyclic oligosaccharide is $\alpha$-cyclodextrin.

10. A cosmetic comprising the oil-in-water type emulsion composition as described in any one of claims 1 to 9.

## INTERNATIONAL SEARCH REPORT

| International application No. |
|---|
| **PCT/JP2023/003838** |

**A. CLASSIFICATION OF SUBJECT MATTER**

*A61K 8/06*(2006.01)i; *A61K 8/60*(2006.01)i; *A61K 8/73*(2006.01)i
FI: A61K8/73; A61K8/60; A61K8/06

According to International Patent Classification (IPC) or to both national classification and IPC

**B. FIELDS SEARCHED**

Minimum documentation searched (classification system followed by classification symbols)

A61K8/73; A61K8/06; A61K8/60

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/230238 A1 (SHOWA SANGYO CO., LTD.) 19 November 2020 (2020-11-19) claims 1-9, paragraphs [0008]-[0010], [0018], [0033], [0042], each example | 1-7 |
| A | | 8-10 |
| Y | JP 2010-226988 A (SHOWA SANGYO CO., LTD.) 14 October 2010 (2010-10-14) claims 1-7, paragraphs [0011]-[0018], [0032]-[0033], each example | 1-7 |
| A | | 8-10 |
| Y | JP 2009-124994 A (AKITA PREFECTURAL UNIV) 11 June 2009 (2009-06-11) claims 1-7, paragraphs [0022], [0025], [0028], each example, fig. 1, 2 | 1-7, 10 |
| A | | 8-9 |
| Y | WO 2019/235142 A1 (SHOWA SANGYO CO., LTD.) 12 December 2019 (2019-12-12) claims 1-12, paragraphs [0048]-[0051], each example | 1-7, 10 |
| A | | 8-9 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 April 2023** | **18 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2023/003838**

### C. DOCUMENTS CONSIDERED TO BE RELEVANT

| Category* | Citation of document, with indication, where appropriate, of the relevant passages | Relevant to claim No. |
|---|---|---|
| Y | JP 2012-135254 A (MIYOSHI OIL & FAT CO LTD) 19 July 2012 (2012-07-19) paragraphs [0019]-[0027], example 5 | 1-7 |
| A | | 8-10 |
| Y | JP 2014-233272 A (Q P CORP) 15 December 2014 (2014-12-15) paragraphs [0073]-[0075], example 9 | 1-7 |
| A | | 8-10 |
| Y | JP 2014-233273 A (Q P CORP) 15 December 2014 (2014-12-15) paragraphs [0072]-[0074], example 8 | 1-7 |
| A | | 8-10 |
| Y | JP 2008-149218 A (KAO CORP) 03 July 2008 (2008-07-03) paragraphs [0045]-[0047], examples 1, 2, paragraph [0048] | 1-7, 10 |
| A | | 8-9 |
| Y | JP 2014-73991 A (FANCL CORP) 24 April 2014 (2014-04-24) paragraphs [0016], [0049]-[0055] | 1-7, 10 |
| A | | 8-9 |

Form PCT/ISA/210 (second sheet) (January 2015)

INTERNATIONAL SEARCH REPORT
Information on patent family members

International application No.

**PCT/JP2023/003838**

| Patent document cited in search report | | | Publication date (day/month/year) | Patent family member(s) | | | Publication date (day/month/year) |
|---|---|---|---|---|---|---|---|
| WO | 2020/230238 | A1 | 19 November 2020 | (Family: none) | | | |
| JP | 2010-226988 | A | 14 October 2010 | (Family: none) | | | |
| JP | 2009-124994 | A | 11 June 2009 | (Family: none) | | | |
| WO | 2019/235142 | A1 | 12 December 2019 | CN | 112292042 | A | |
| JP | 2012-135254 | A | 19 July 2012 | (Family: none) | | | |
| JP | 2014-233272 | A | 15 December 2014 | (Family: none) | | | |
| JP | 2014-233273 | A | 15 December 2014 | (Family: none) | | | |
| JP | 2008-149218 | A | 03 July 2008 | (Family: none) | | | |
| JP | 2014-73991 | A | 24 April 2014 | (Family: none) | | | |

Form PCT/ISA/210 (patent family annex) (January 2015)

**EP 4 477 211 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021052739 A
- JP 2021122269 A
- JP 2021019525 A
- JP 2004315481 A
- JP 2005213176 A

**Non-patent literature cited in the description**

- *Eur. J. Biochem*, 1975, vol. 59, 615-625
- Starch and Sugar Related Industry Analysis Method. Sugar Technology Committee